# EUROPEAN PATENT APPLICATION

(11) **EP 1 419 767 A1**
(43) Date of publication of application: **19.05.2004**
(21) Application number: 02762807.2
(22) Date of filing: 19.08.2002
(51) Int. Cl.: A61K 31/166, A61P 9/00, A61P 9/04, A61P 43/00

(54) **REMEDIAL AGENT FOR CARDIAC FAILURE**

(30) Priority: 20.08.2001 JP 2001248545
(71) Applicant: ONO PHARMACEUTICAL CO., LTD., Osaka-shi, Osaka 541-8526 (JP)
(72) Inventor: KIHARA, Yasuki, Ohtsu-shi, Shiga 520-0011 (JP)
(74) Representative: Henkel, Feiler & Hänzel
(86) International application number: PCT/JP2002/008346
(87) International publication number: WO 2003/015762

(57) **Abstract**

A therapeutic and/or prophylactic drug for cardiac failure and cardiac hypertrophy, comprising a derivative of hydroxamic acid, which is represented by the formula (I) 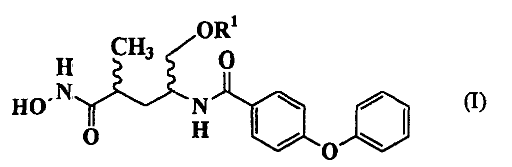 (wherein R¹ represents a halogen atom, C1-8 alkyl, or C1-8 alkyl substituted with -OR², in which R² presents a halogen atom, C1-8 alkyl, benzil, or C1-8 alkyl substituted with C1-8 alkoxy(s)), or a non-toxic salt thereof.

This inventive compound represented by the formula (I) has the inhibitory effect of matrix metalloproteinase, and is effective to cardiac failure and cardiac hypertrophy.

## Description

### TECHNICAL FIELD

The present invention relates to a medicine for cardiac failure and cardiac hypertrophy.

More particularly, the present invention relates to therapeutic and/or prophylactic drugs for cardiac failure and cardiac hypertrophy, comprising derivatives of hydroxamic acid, which are represented by the formula (I) 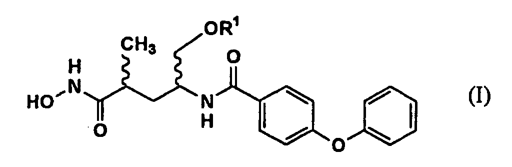 (all signs in the formula represent the same meanings as postscripts.) with inhibitory activities for matrix metalloproteinases or these non-toxic salts, as active ingredients.

### BACKGROUND OF THE INVENTION

Matrix metalloproteinases (hereafter, it is abbreviated with MMP.) are neutral metalloproteinases that have zinc (hereafter, it is abbreviated with Zn²⁺.) in the active center. So far, at least, the molecular species more than 10 species with different primary structure have been identified. Concretely, interstitial collagenase (MMP-1), neutrophil collagenase (MMP-8), collagenase-3 (MMP-13), gelatinase A (MMP-2), gelatinase B (MMP-9), stromelysin-1 (MMP-3), stromelysin-2 (MMP-10), matrilysin (MMP-7), metalloelastase (MMP-12), etc. are enumerated.

Under physiological condition, they are effective on growth and tissue remodeling of articulation tissue, bone tissue and connective tissue, etc. by degrading collagen, laminin, proteoglycans, fibronectin, elastin, gelatin, etc. However, it is consider that disorganization of various tissues under morbid condition was caused by increase in expressions or activities of MMPs, which was caused by failure of MMP modulation.

For example, an increase in MMP activity of left ventricle muscle is confirmed in dilated cardiomyopathy [Circulation, 97(17), 1708-15 (1998)]. By a model of congestive heart failure, it has been proved that a change in MMP enzyme activity and the protein content in left ventricle could cause the beginning and the progress of left ventricular enlargement and the dysfunction [Circ Res, 82(4), 482-95 (1998)].

On the other hand, normal cardiac output can't be maintained by cardiac disturbance of cardiac failure, the causes are various such as cardiac infarct, hypertensive heart disease, and enlarged heart, etc.

For a decrease in cardiac output that is happened by chronic mechanical load to heart, heart is adapted by morphologic changes such as the enlargement and the hypertrophy of ventricles and the compensative mechanism such an increase, etc. in sympathetic nerve activity to keep systolic stress at acute stage to be normal. For further information, various nerve and humoral factors are mobilized for mechanical loading, and the protein synthesis of myocardial cellular increases. Further, changes in expression of various genes and qualitative changes in non-cardiac muscle cells, for example, increased synthesis, etc. of extracellular matrix are admitted. However, there is a negative aspect of adding load to heart when compensation mechanism to the load continues long. In addition, by failure of compensation mechanism, a ventricle of hypertrophic heart expands, the cardiac performance decreases, and the heart shifts to ventricular failure. Like this, it is regarded that cardiac hypertrophy is an adjustment phenomenon to load, and the former stage of ventricular failure. Therefore, if compensation failure from cardiac hypertrophy to ventricular failure and cellular factors can be clarified, and pharmacologically controlled, it is possible to use them as a medical treatment of cardiac failure.

Now, in the treatment of ventricular failure, there are improvement of pump action, reduction of load, and improvement of oedema, cardiotomics, inhibitors of angiotensin converting enzyme, and diuretic drugs, etc. are used, respectively. Since these have the problem in the margin of safety and the side effect, and the number of patients in which the effect is achieved is statistically only parts, these are not satisfying still enough.

For example, there is a specification of WO99/32150 as a patent application that enumerats ventricular failure as adaptive disease of compounds with MMP inhibitory effect.

And, it is reported that PD166793, which is a MMP inhibitor, could be effective on animal test mode of ventricular failure [J Pharmacol Exp Ther, 291(2), 799-811 (1999), Circ Res., 85(4), 364-376 (1999)]. However, there is no treatment and/or prophylactic medicine for ventricular failure and cardiac hypertrophy, comprising compounds with MMP inhibitory effect now.

### DISCLOSURE OF THE INVENTION

As previously described, it was eagerly looking forward to supply potent therapeutic and/or prophylactic agents for cardiac failure and cardiac hypertrophy, and expected that MMP inhibitors will become the therapeutic and/or prophylactic agents.

However, it is already natural that all known MMP inhibitory agents are not necessarily effective in cardiac failure and cardiac hypertrophy, and it is not achieved still.

Under the above condition, as a result that the present inventors examined assiduously, they first certified that derivatives of hydroxamic acid, which are represented by the formula (I), or the non-toxic salts could be useful for cardiac failure and cardiac hypertrophy, and accomplished the present invention.

The present invention relates to therapeutic and/or prophylactic drugs for cardiac failure and cardiac hypertrophy, comprising derivatives of hydroxamic acid, which are represented by the formula (I), 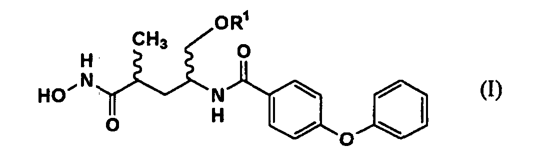 (wherein R¹ represents a halogen atom, C1-8 alkyl, or C1-8 alkyl substituted with -OR², in which R² presents a halogen atom, C1-8 alkyl, benzil, or C1-8 alkyl substituted with C1-8 alkoxy(s).) or the non-toxic salts.

The derivatives of hydroxamic acid, which are represented by the formula (I), have been described in a specification of WO99/19296 as compounds with MMP inhibitory activity.

In the above specification, it have been described that the derivatives of hydroxamic acid, which are represented by the formula (I), have MMP inhibitory activity, so they are useful for treatment and/or prevention of diseases, for example, rheumatoid diseases, arthrosteitis, unusual bone resorption, osteoporosis, periodontitis, interstitial nephritis, arteriosclerosis, pulmonary emphysema, cirrhosis, cornea injury, cornea ulcer, metastasis, invasion or growth of tumor cells, autoimmune disease (Crohn's disease, Sjogren's syndrome), disease caused by vascular emigration or infiltration of leukocytes, arterialization, multiple sclerosis, arota aneurysm, endometriosis. However, there is no description that the compounds represented by the formula (I) are useful for cardiac failure and cardiac hypertrophy.

In spite of some implications like this, when some MMP inhibitors of which the effects on cardiac failure and cardiac hypertrophy aren't actually known were examined by a model of actual heart failure, the present inventor first found that the compounds represented by the formula (I) could be effective.

### DETAILED DESCRIPTION OF THE INVENTION

C1-8 alkyl in the formula (I) representing compounds of hydroxamic acid of the present invention represents methyl, ethyl, propyl, butyl, pentyl, hexyl, heptyl, octyl group, or these isomers.

In the formula (I), C1-8 alkyl substituted with -OR² represents methyl, ethyl, propyl, butyl, pentyl, hexyl, heptyl, or octyl group, which were substituted with a single -OR², or these isomers.

In the formula (I), C1-8 alkyl substituted with C1-8 alkoxy groups represents methoxy, ethoxy, propoxy, butoxy, pentyloxy, hexyloxy, heptyloxy, or octyloxy group; methyl, ethyl, propyl, butyl, pentyl, hexyl, heptyl, octyl group, or these isomers, which are substituted with a group selected from these isomers.

In the formula (I), 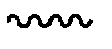 sign represents a bond that shows an isomer with asymmetric carbon or the mixture, concretely, 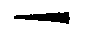 sign represents a bind forward space, and 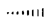 sign represents a bind backward space, or these mixture.

In the present invention, isomers contain all isomers as long as it does not especially direct it. For example, straight chains and branched chains are contained in alkyl and alkoxy group. In addition, isomers with asymmetric carbons, etc. (R, S body, α, β body, enantiomer, and diastereomer), optical isomers with optical rotation (D, L, d, and I body), polarity bodies separated by chromatograph (high polarity body and low polarity body), balanced compounds, these arbitrary ratio compounds, and racemic mixtures are contained in the present invention.

All groups represented by R¹ are suitable for R¹ in the formula (I) prefers, more suitably, a hydrogen atom, C1-4 alkyl, or C1-4 alkyl substituted with a single -OR².

All groups represented by R² are suitable for R² in the formula (I), more suitably, a hydrogen atom, C1-4 alkyl, benzil, or C1-4 alkyl substituted with a single C1-4 alkoxy.

As concrete compounds used for the present invention,
N-hydroxy-5-hydroxy-2(S)-methyl-4(S)-(4-phenoxybenzoyl)aminopentanamide,
N-hydroxy-5-methoxymethyloxy-2(S)-methyl-4(S)-(4-phenoxybenzoyl)aminopentanamide,
N-hydroxy-5-ethoxymethyloxy-2(S)-methyl-4(S)-(4-phenoxybenzoyl)aminopentanamide,
N-hydroxy-5-ethoxymethyloxy-2(R)-methyl-4(R)-[N-(4-phenoxyphenylcarbonyl)amino]pentanamide,
N-hydroxy-5-ethoxymethyloxy-2(R)-methyl-4(S)-[N-(4-phenoxyphenylcarbonyl)amino]pentanamide,
N-hydroxy-5-benzyloxymethyloxy-2(S)-methyl-4(S)-(4-phenoxybenzoyl)aminopentanamide,
N-hydroxy-5-(2-methoxyethoxy)methyloxy-2(S)-methyl-4(S)-(4-phenoxybenzoyl)aminopentanamide, or non-toxicity salts thereof are enumerated.

The compounds used in the present invention may be used as the following, non-toxic salts, which non-toxic and water-soluble one are suitable.

As suitable salts, alkaline earth salts (potassium, sodium, etc.), alkaline earth metal salts (calcium, magnesium, etc.), ammonium salts, pharmaceutically acceptable organic amine salts (tetramethylammonium, triethylamine, methylamine, dimethylamine, cyclopentylamine, benzylamine, phenethylamine, piperidine, monoethanolamine, diethanolamine, tris(hydroxymethyl)amine, lysine, arginine, N-methyl-D-glucamine, etc.) are enumerated.

The compounds or the non-toxic salts used in the present invention may be converted into the hydrate by a conventional manner.

### BRIEF DESCRIPTION OF DRAWING

Figure 1 shows cumulative survival rates of rats when administering N-hydroxy-5-ethoxymethyloxy-2(S)-methyl-4(S)-(4-phenoxybenzoyl)aminopentanamide (compound (II)) to Dahl's salt-sensitive rats were continued after development of cardiac hypertrophy until dying.
Figures 2-5 show left ventricular end-diastolic dimension (LVDD) on 11, 15, and 17 weeks by using transthoracic echocardiography (figure 2) , left ventricular end-systolic dimension (LVDS) (figure 3), posterior wall thickness (PWT) (figure 4), wall stress (figure 5), respectively when administering compound (II) to Dahl's salt-sensitive rats were continued from 11 or 15 weeks of ages after development of cardiac hypertrophy until dying.
Figure 6 shows measurement results as for internal pressure (mmH₂O) and volume of left ventricle (LV) in hearts of Dahl's salt-sensitive rat to which compound (II) have been administered from 11 or 15 weeks after development of cardiac hypertrophy until dying.

### BEST FORM TO EXECUTE INVENTION

Efficiencies of the compounds in the present invention on cardiac failure and cardiac hypertrophy was proven by the following experiments.
The model of cardiac failure using Dahl's salt-sensitive rat
N-hydroxy-5-ethoxymethyloxy-2(S)-methyl-4(S)-(4-phenoxybenzoyl) aminopentanamide (hereafter, abbreviated with compound (II)) represented by the formula (II) 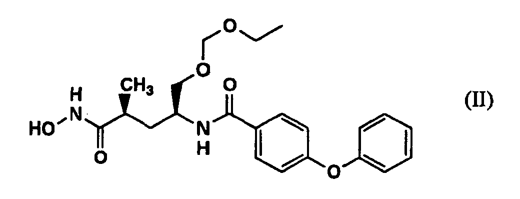 as a subject drug was used.

### Experimental method

### (1) Life prolongation

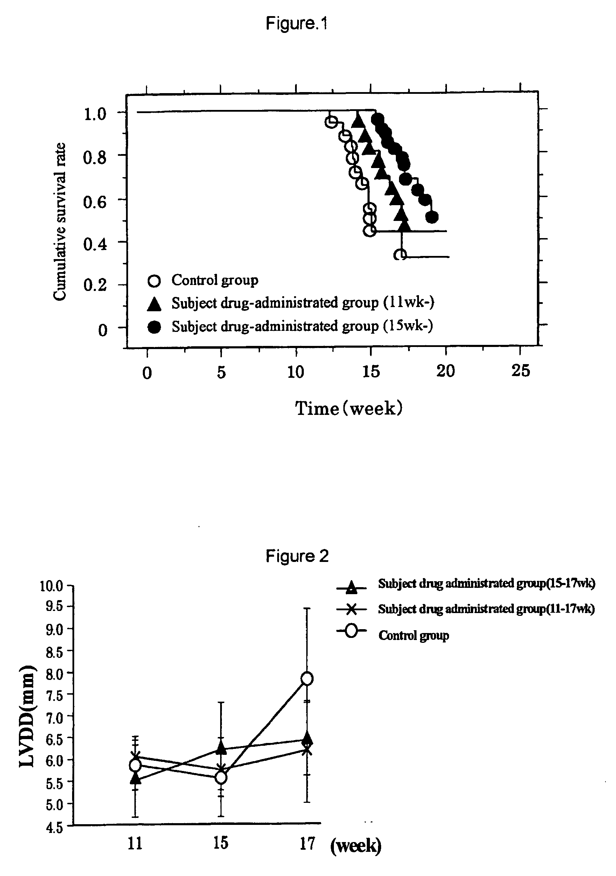

A subject drug (100 mg/kg) was administered to Dahl's salt-sensitive rat (10 examples in each crowd) bred with high salt diet twice a day from 11 or 15 weeks after development of cardiac hypertrophy until dying. Administering was continued after development of cardiac hypertrophy until dying. For administering and afterwards, each survive of the control group (vehicle) (0.5% carboxymethylcellulose administrated group) and the subject drug administrated group was confirmed respectively, and each accumulation survival rates was calculated by the analysis using the Kaplan-Meier method. Figure 1 shows the result.

### (2) Measurement of left heart function and form

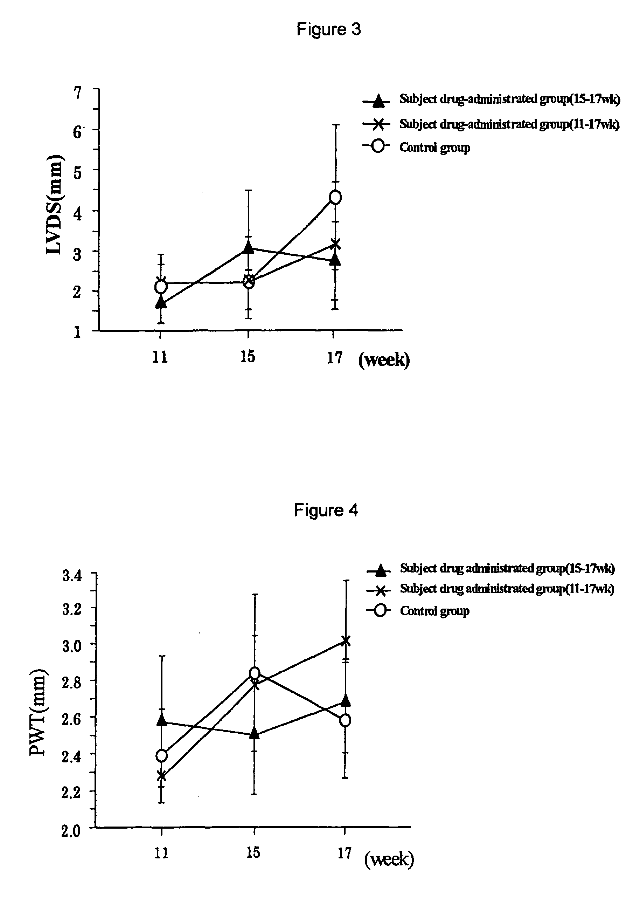

In the above model of cardiac failure using Dahl's salt-sensitive rat, left ventricular end-diastolic dimension (LVDD) on 11, 15, and 17 weeks by using transthoracic echocardiography, left ventricular end-systolic dimension (LVDS), posterior wall thickness (PWT), wall stress were measured. Figures 2-5 show these results.

### (3) P-V curve

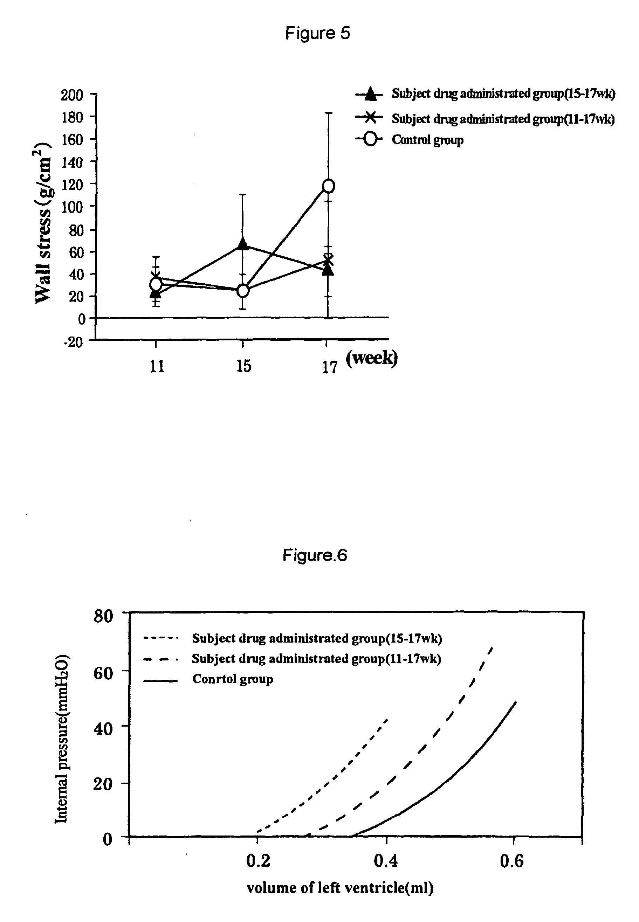

In the above model of cardiac failure using Dahl's salt-sensitive rat, internal pressure and volume of left ventricle of hearts removed from 11, 15, and 17 weeks rats were measured under perfuse by Langendorff's method. Figure 6 shows the result.

### Effect of invention

In this model, the compounds represented by the formula (I) showed effects on rat's life prolongment compared with the control group.

Since LVDD and LVDS have decreased on 17 weeks of ages, it has been understood that making left ventricular lumen narrow has happened. In addition, because of increase in PWT with decrease in LVDD, it has been understood that thickening of wall with making left ventricular lumen narrow and decrease in wall stress have happened. As these results, it can be considered that the compound represented by the formula (I) could suppress stretching of left ventricular seen under ventricular failure.

Since P-V curve of the subject drug administrated group shifts to the left side compared with one of the control group, and the inclination is steep, it is considered that diameter of left ventricular could become small, and left ventricular become hardening. The decrease in volume of left ventricular correlates to the above decrease in LVDD and LVDS.

Therefore, it can be considered that the compound represented by the formula (I) could be effective to cardiac failure or cardiac hypertrophy.

### Toxicity

It was considered that the toxicities of the compounds in the present invention could be very low and safe to use as a medicine. For example, the lowest lethal dose in oral administration of compound (II) to mice was 2000 mg/kg.

### Application for Pharmaceuticals

The compounds used in the present invention, which are hydroxamic acid derivatives represented by the formula (I) or the non-toxic salts with an inhibitory activities of matrix metalloproteinases, for example, gelatinase, stromelysin, or collagenase, etc., are useful for treatment and/or prevention for cardiac failure and cardiac hypertrophy in animals including human, especially human.

The compounds represented by the formula (I) or the non-toxicity salts,
1) to supplement and/or reinforce the therapeutic and/or prophylactic effect of the compounds,
2) to improve the movement and absorption of the compounds, and to decrease the dosage, and/or
3) to reduce the side effect of the compounds, may be administered as a combined drug combining with other medicines.

Other concomitant drugs with the compounds represented by the formula (I) and other medicines may be administered in the combined form that mixes both elements in the formulation or in the form administered as separate formulation. In the form administered as separate formulation, the different and simultaneous administration are included. In the different administration, the compounds represented by the formula (I) may be previously administered, followed by other medicines, or other medicines may be previously administered, followed by the compounds represented by the formula (I). Each medication method may be different or same.

Illnesses that the therapeutic and/or prophylactic effects of the above other concomitant drugs can be expected, which only has to be one that the therapeutic and/or prophylactic effects of the compounds represented by the formula (I) is/are supplemented and/or reinforced, are not limited especially.

As other medicines to supplement and/or reinforce the therapeutic and/or prophylactic effects of the compounds represented by the formula (I) for cardiac failure and cardiac hypertrophy, for example, diuretic drugs, cardiac glycosides, calcium antagonists, peripheral vasodilators, alpha-adrenergic blocking agents, nitrous acid medicines, inhibitors of angiotensin converting enzyme, autonomic ganglionic blockers, and catecholamines, etc. are enumerated.

As diuretics, for example, mannitol, furosemide, acetazolamide, diclofenamide, methazolamide, trichlormethiazide, mefruside, spironolactone, and aminophylline, etc. are enumerated. As cardiac glycosides, digitoxin, digoxin, deslanoside, methyldigoxin, proscillaridin, strophanthin, and lanatoside C, etc. are enumerated.

As calcium antagonist, nifedipines, benidipine hydrochloride, diltiazem hydrochloride; verapamil hydrochloride, nisoldipine, nitrendipine, bepridil hydrochloride, amlodipine besilate, and lomerizine hydrochloride, etc. are enumerated.

As inhibitors of angiotensin converting enzyme, alacepril, imidapril hydrochloride,quinapril hydrochloride, temocapril hydrochloride, delapril hydrochloride, benazepril hydrochloride, captopril, trandolapril, perindopril erbumine, enalapril maleate, and lisinopril, etc. are enumerated.

As catecholamines, norepinephrine, isoproterenol, dopamine, denopamine, and kalgut are enumerated.

Mass ratio of the compounds represented by the formula (I) to other medicines is not especially limited.

Other medicines may be administered combining with two arbitrary kinds or more.

And, in other medicines that supplement and/or reinforce therapeutic and/or prophylactic effects by the compounds represented by the formula (I), not only one found by present based on the above mechanism but also the one that will be found in the future are contained.

To use the compounds represented by the formula (I) or Other concomitant drugs containing the compounds represented by the formula (I) and other medicines by the above purpose, they are usually administered systemically or locally, and orally or parenterally.

The applied doses are determined depending upon, for example, age, body weight, symptom, the desired therapeutic effect, the route of administration, and the duration of the treatment. Generally, a dose from 1 mg to 1000 mg per an adult is orally administrated from once to several times per day, parenterally from 0.1 mg to 100 mg, or intravenously from 1 to 24 hours per day, continuously.

As above mentioned, because the dose depends upon various conditions, there are cases in which the dose is lower or higher than the above dose.

When the compounds represented by the formula (I) or other concomitant drugs containing them and other medicines are administered, they are used as solid medicines and liquid medicines for internal use, and injections, liquid medicines, external preparations, suppositoriums, eye drops, and inhalants, etc. for parenteral administration.

Solid forms for oral administration may include compressed tablets, pills, capsules, dispersible powders, and granules, etc. Capsules may include hard capsules and soft capsules.

In such solid forms for oral administration, one or more of active compound(s) may be admixed itself (themselves) or with vehicles (lactose, mannitol, glucose, microcrystalline cellulose, starch, etc.), binders (hydroxypropyl cellulose, polyvinylpyrrolidone or magnesium metasilicate aluminate, etc.), disintegrants (ellulose calcium glycolate, etc.), lubricants (magnesium stearate, etc.), stabilizers, and/or solubilizers (glutamic acid or aspartic acid, etc.) and used manufacturing pharmaceutically according to well known methods. If desired, they may be coated with coating agents (sugar, gelatin, hydroxypropyl cellulose or hydroxypropylmethyl cellulose phthalate, etc.) or coated with two or more films. Further, they may include capsules comprising absorbable materials such as gelatin.

Liquid forms for oral administration may include pharmaceutically acceptable solutions, suspensions, emulsions, syrups and elixirs, etc. In such as forms, one or more of active compound(s) may be dissolved, suspended or emulsified into diluent(s) (such as purified water, ethanol or a mixture thereof) generally used. Besides this liquid forms may also comprise wetting agent, suspending agent, emulsifying agent, sweetening agent, flavoring agent, aroma, preservatives, or buffer, etc.

In external preparations for parenteral administration, for example, ointment, gel, cream, fomentation, patch, liniment, aerosol, inhalant, spray, aerosol, and nasal drop, etc. are contained. They contain one or any more activators, and are prepared by a well-known method or usually used prescription.

Ointments are manufactured by a well-known or usually used prescription. For example, they are made by incorporating or melting one or more activators to the base. Ointment bases are chosen from the well-known or usually used one. They are used mixing a single or two kinds or more chosen from, for example, higher fatty acid or higher fatty acid ester (adipic acid, myristic acid, palmitic acid, stearic acid, oleic acid, adipic acid ester, myristic acid ester, retinol palmitate, stearic acid ester, and oleic acid ester, etc.), wax (wax, spermaceti, and ceresin, etc.), surfactant (polyoxyethylene alkylether phosphate ester, etc.), higher alcohol (cetanol, stearyl alcohol, and cetostearyl alcohol, etc.), silicone oil (dimethylpolysiloxane), hydrocarbon (hydrophilic petrolatum, white petrolatum, purified lanolin, and liquid paraffin, etc.), glycol (ethylene glycol, diethylene glycol, propylene glycol, polyethylene glycol, and macrogol, etc.), vegetable oil (castor oil, olive oil, sesame oil, and turpentine oil, etc.), animal oil (mink oil, yolk oil, squalane, and squalene, etc.), water, absorption promoter, and rash inhibitor. In addition, they may contain moisturizing agent, preservative, stabilizer, antioxidant, and flavor, etc.

Gels are manufactured by a well-known or usually used prescription. For example, they are manufactured by melting one or more activators to base. Gel bases are chosen from the well-known one or the one usually used one. They are used mixing single or two kinds or more chosen from, for example, lower alcohol (ethanol and isopropyl alcohol, etc.), gelatinizer (carboxymethylcellulose, hydroxyethyl cellulose, hydroxypropylcellulose, and ethyl cellulose, etc.), neutralizer (triethanolamine and diisopropanolamine, etc.), surfactant (polyethyleneglycol monostearate, etc.), gums, water, absorption promoter, and rash inhibitor. In addition, they may contain preservative, antioxidant, and flavor, etc.

Creams are manufactured by a well-known or usually used prescription. For example, they are manufactured by melting one or more activators to base and emulsifying. Creams are chosen from the well-known or usually used one. They are used mixing single or two kinds or more chosen from, for example, higher fatty acid ester, lower alcohol, hydrocarbons, polyhydric alcohol (propylene glycol, 1, 3-butylene glycol, etc.), higher alcohol 2-hexyldecanol and cetanol, etc.), emulsify (polyoxyethylene alkyl ether and fatty acid ester, etc.), water, absorption promotor, and rash inhibitor. In addition, they may contain preservative, antioxidant, and flavor, etc.

Fomentations are manufactured by a well-known or usually used prescription. For example, they are manufactured by melting one or more activators to base, kneading and roll spreading on support medium. Compress bases are chosen from the well-known or usually used one. They are used mixing single or two kinds or more chosen from, for example, thickener (polyacrylic acid, polyvinylpyrrolidone, acacia, starch, gelatin, and methylcellulose, etc.), humectants (urea, glycerin, and propylene glycol, etc.), filler (china clay, zinc oxide, talc, calcium, and magnesium, etc.), water, solubilizer, tackifier, and rash inhibitor. In addition, they may contain preservative, antioxidant, and flavor, etc.

Patchs are manufactured by a well-known or usually used prescription. For example, they are manufactured by melting one or more activators to base, kneading and roll spreading on support medium. Bases for patch are chosen from well-known or usually used one. For example, one or two kinds or more chosen from high molecular group base, fats and oils, higher fatty acid, tackifier, and rash inhibitor is/are used by mixing. In addition, they may contain preservative, antioxidant, and flavor, etc.

Liniments are manufactured by a well-known or usually used prescription. For example, one or more activators is/are manufactured by being dissolved, suspended or emulsified to one or two kinds or more chosen from water, alcohol (ethanol and polyethyleneglycol, etc.), higher fatty acid, glycerin, soap, or emulsify, etc. In addition, they may contain preservative, antioxidant, and flavor, etc.

Aerosols, inhalants, and aerosols may include stabilizers such as sodium hydrogen sulfite besides diluent usually used and isotonicity medicine like buffer giving isotonicity, for example, sodium chloride, sodium citrate, or citrate. The manufacturing method of aerosol has been described in detail, for example, in U.S. patent No.2,868,691 and No.3,095,355.

Injections for parenteral administration may include solution, suspension, emulsion, and solid injections which are used dissolving or suspending in solvent for time of use. The injections are used dissolving, suspending, and emulsifying one or more of active compound(s) to solvent. As solvent, distilled water for injection, physiological salt solution, vegetable oil, propylene glycol, polyethylene glycol, alcohols like ethanol, or their mixtures may be used. Further, these injections may contain stabilizer, solubilizer (glutamic acid, aspartic acid or POLYSORBATE80 (registered trade mark), etc.), suspending agent, emulsifying agent, soothing agent, buffer, preservative, etc. These may be sterilized at a final step or prepared by aseptic manipulation. These may also be used by being manufactured as sterile solid forms, for example, freeze-dried products and dissolved in aseptic or sterile water or other sterile diluent(s) for injection immediately before using.

Inhalants for parenteral administration may include aerosol agent, inhalant powder or inhalant liquid, which may be used by being dissolved or suspended in water or other suitable media before using.

Inhalants are manufactured based on a well-known method.

For example, inhalant liquids are prepared properly selecting, if necessary, preservative (benzalkonium chloride and paraben, etc.), coloring agent, buffer (sodium phosphate and sodium acetate, etc.), tonicity agent (sodium chloride and concentrated glycerin, etc.), thickener (carboxyvinyl polymer, etc.), and absorption enhancers, etc.

Inhalant powders are prepared properly selecting, if necessary, lubricant (stearic acid and the salt, etc.), binder (starch and dextrin, etc.), filler (lactose and cellulose, etc.), coloring agent, preservative (benzalkonium chloride and paraben, etc.), and absorption enhancer, etc.

When inhalant liquids are administered, sprayer (atomizer and nebulizer) is usually used, and when inhalant powder are administered, an inhalation administering machine for powder is usually used.

Pessaries, etc., for a suppository and intravaginal administration to intrarectally administrate with common procedure, which contains one or more activators are included as other compositions for parenteral administration.

### Formulation example 1:

The following components were admixed in conventional method and punched out to obtain 100 tablets each containing 50 mg of active ingredient in a tablet.
- N-hydroxy-5-ethoxymethyloxy-2(S)-methyl-4(S)-(4-phenoxybenzoyl) aminopentanamide 5.0 g
- Carboxymethylcellulose calcium (disintegrator) 0.2 g
- Magnesium stearate (lubricant) 0.1 g
- Crystallite cellulose 4.7g

### Formulation example 2:

After the following components were admixed in conventional method, the solution was sterilized in conventional manner, placed 5 ml portions into ampoules and freeze-dried to obtain 100 ampoules each containing 20 mg of the active ingredient.
- N-hydroxy-5-ethoxymethyloxy-2(S)-methyl-4(S)-(4-phenoxybenzoyl) aminopentanamide 2.0 g
- mannitol 20 g
- distilled water 500 ml

## Claims

1. A therapeutic and/or prophylactic drug for cardiac failure and/or cardiac hypertrophy comprising a derivative of hydroxamic acid, which is represented by the formula (I) 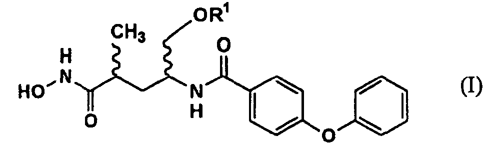 wherein R¹ represents a halogen atom, C1-8 alkyl, or C1-8 alkyl substituted with -OR², in which R² presents a halogen atom, C1-8 alkyl, benzil, or C1-8 alkyl substituted with C1-8 alkoxy(s), or a non-toxic salt thereof.

2. The therapeutic and/or prophylactic drug for cardiac failure and cardiac hypertrophy, comprising the compound of claim 1, wherein the compound is
(1) N-hydroxy-5-hydroxy-2(S)-methyl-4(S)-(4-phenoxybenzoyl)aminopentanamide,
(2) N-hydroxy-5-methoxymethyloxy-2(S)-methyl-4(S)-(4-phenoxybenzoyl)aminopentanamide,
(3) N-hydroxy-5-ethoxymethyloxy-2(S)-methyl-4(S)-(4-phenoxybenzoyl)aminopentanamide,
(4) N-hydroxy-5-ethoxymethyloxy-2(R)-methyl-4(R)-[N-(4-phenoxyphenylcarbonyl)amino]pentanamide,
(5) N-hydroxy-5-ethoxymethyloxy-2(R)-methyl-4(S)-[N-(4-phenoxyphenylcarbonyl)amino]pentanamide,
(6) N-hydroxy-5-benzyloxymethyloxy-2(S)-methyl-4(S)-(4-phenoxybenzoyl)aminopentanamide,
(7) N-hydroxy-5-(2-methoxyethoxy)methyloxy-2(S)-methyl-4(S)-(4-phenoxybenzoyl)aminopentanamide, or a non-toxicity salt thereof.
